# EUROPÄISCHE PATENTANMELDUNG

(11) **EP 2 201 968 A1**
(43) Veröffentlichungstag der Anmeldung: **30.06.2010**
(21) Anmeldenummer: 08022458.7
(22) Anmeldetag: 24.12.2008
(51) Int. Cl.: A61M 5/158, A61M 5/142, A61M 5/32

(54) **Insertionssystem und Insertionsvorrichtung**

(71) Anmelder: Roche Diagnostics GmbH, 68305 Mannheim (DE); F.HOFFMANN-LA ROCHE AG, 4070 Basel (CH)
(72) Erfinder: Kube, Oliver, 67549 Worms (DE)
(74) Vertreter: Twelmeier Mommer & Partner

(57) **Zusammenfassung**

Die Erfindung betrifft ein Insertionssystem mit einer Basiseinheit (2) zum Aufsetzen auf den Körper eines Patienten und einer an die Basiseinheit (2) ankoppelbaren Insertionsvorrichtung (1), wobei die Insertionsvorrichtung (1) einen Insertionsnadelhalter (7) zum Halten einer Insertionsnadel (8) und einen Antriebsmechanismus zum Bewegen des Insertionsnadelhalters (7) in einer Stichrichtung aufweist. Erfindungsgemäß ist vorgesehen, dass die Insertionsvorrichtung (1) einen Sperrmechanismus (10) aufweist, der in einem aktiven Zustand eine Sperrung des Antriebsmechanismus bewirkt und durch Ankoppeln der Insertionsvorrichtung (1) an die Basiseinheit (2) in einen inaktiven Zustand versetzt wird, in dem die Sperrung gelöst ist.

## Beschreibung

Die Erfindung betrifft ein Insertionssystem mit den im Oberbegriff des Anspruchs 1 angegebenen Merkmalen sowie eine entsprechende Insertionsvorrichtung.

Um Sensoren zur Messung von Analytkonzentrationen in vivo, beispielsweise der Glukosekonzentrationen, in Körpergewebe eines Patienten zu insertieren, beispielsweise in Unterhautfettgewebe, werden Insertionsvorrichtungen verwendet, die mit einem Antriebsmechanismus eine Stichbewegung einer Insertionsnadel bewirken. Hierfür gebräuchliche Insertionsnadeln sind als Hohlnadeln oder V-förmige Rinnen ausgebildet, in denen ein Sensor liegt. Der Sensor kann beispielsweise als ein Elektrodensystem für elektrochemische Messungen ausgebildet sein oder einen mikrofluidischen Katheter zum Ein- und Ausleiten einer Perfusionsflüssigkeit umfassen. Nach einem Einstich wird die Insertionsnadel aus dem Körpergewebe herausgezogen, wobei der Sensor in der erzeugten Stichwunde verbleibt.

Eine weitere Anwendung von Insertionsvorrichtungen ist beispielsweise die Applikation von Kathetern, insbesondere zur Infusion von Insulin oder anderen Wirkstoffen.

Derartige Insertionsvorrichtungen bilden zusammen mit einer Basiseinheit, an die sie für eine Insertion angekoppelt werden können, ein Insertionssystem. Basiseinheiten werden üblicherweise auf den Körper eines Patienten aufgeklebt. Anschließend kann eine Insertionsvorrichtung an die Basiseinheit angekoppelt werden. Nach abgeschlossener Insertion kann die Insertionsvorrichtung von der Basiseinheit abgekoppelt werden, so dass die Basiseinheit beispielsweise als Träger oder Anschlusselement eines insertierten Sensors oder Katheters auf dem Körper des Patienten verbleibt.

Insertionssysteme werden häufig von den Patienten selbst bedient, beispielsweise um Katheter zum Anschließen an eine Insulinpumpe oder Sensoren zur Messung der Glukosekonzentration zu Insertieren. Bei der Entwicklung von derartigen Insertionssystemen ist es deshalb ein ständiges Ziel, dass diese möglichst einfach und sicher bedient werden können.

Diese Aufgabe wird durch ein Insertionssystem mit den im Anspruch 1 angegebenen Merkmalen sowie eine Insertionsvorrichtung für ein solches Insertionssystem gemäß Anspruch 2 gelöst. Vorteilhafte Weiterbildungen der Erfindung sind Gegenstand von Unteransprüchen.

Erfindungsgemäß ist vorgesehen, dass die Insertionsvorrichtung einen Sperrmechanismus aufweist, der in einem aktiven Zustand eine Sperrung des Antriebsmechanismus bewirkt und durch Ankoppeln der Insertionsvorrichtung an die Basiseinheit in einen inaktiven Zustand versetzt wird, in dem die Sperrung gelöst ist. Mit einem solchen Sperrmechanismus, der in seinem aktiven Zustand den Antriebsmechanismus blockiert, kann ein vorzeitiges Auslösen eines Stichs verhindert und so die Gefahr einer Verletzung bei der Handhabung der Insertionsvorrichtung reduziert werden.

Bei einer erfindungsgemäßen Insertionsvorrichtung entriegelt der Sperrmechanismus automatisch, wenn die Insertionsvorrichtung an die Basiseinheit angekoppelt wird. Erfindungsgemäß lässt sich deshalb erreichen, dass der Sperrmechanismus nur dann entriegelt ist, wenn die Insertionsvorrichtung an die Basiseinheit angekoppelt ist. Vorteilhaft lässt sich also erreichen, dass ein Benutzer den Sperrmechanismus nur durch Ankoppeln der Insertionsvorrichtung an die Basiseinheit entriegeln kann. Ein Stich lässt sich folglich vorteilhaft nur auslösen, wenn die Insertionsvorrichtung an die Basiseinheit angekoppelt ist, so dass die Gefahr einer Verletzung bei fehlerhafter Handhabung weitestgehend ausgeschlossen ist.

Neben einer erhöhten Sicherheit vor Verletzungen hat ein erfindungsgemäßer Sperrmechanismus zudem den Vorteil, dass sich die Bedienbarkeit der Insertionsvorrichtung wesentlich vereinfachen lässt. Während bei bekannten Insertionsvorrichtungen nämlich in der Regel mehr oder weniger aufwendige und komplizierte Auslöse- oder Betätigungsmechanismen verwendet werden, um ein unbeabsichtigtes Auslösen eines Stichs zu verhindern, beispielsweise indem mehrer Betätigungselemente vorgesehen sind, die in einer vorgegebene Reihenfolge oder Kombination betätigt werden müssen, kann bei einem erfindungsgemäßen Insertionssystem auf derartige Maßnahmen verzichtet werden. Da der Antriebsmechanismus nur nach Ankopplung der Insertionsvorrichtung an die Basiseinheit einen Stich bewirken kann, ist selbst bei Einsatz von beliebig einfachen Auslöse- oder Betätigungsmechanismen ein vorzeitiges Auslösen eines Stichs ausgeschlossen.

Ein erfindungsgemäßer Sicherungsmechanismus kann beispielsweise durch Magnetkraft aus seinem aktiven Zustand in seinen inaktiven Zustand versetzt werden. Hierfür erforderliche Magnete können an der Basiseinheit und/oder der Insertionsvorrichtung angebracht sein. Möglich ist es auch, den Sicherungsmechanismus elektrisch zu deaktivieren, indem beim Ankoppeln der Insertionsvorrichtung an die Basiseinheit ein elektrischer Kontakt geschlossen wird. Bevorzugt funktioniert der Sicherungsmechanismus aber rein mechanisch, beispielsweise, indem an der Basiseinheit ein Indexstift vorgesehen ist, der beim Ankoppeln den Sicherungsmechanismus betätigt und dabei in den inaktiven Zustand versetzt.

Ein erfindungsgemäßer Sperrmechanismus kann beispielsweise mit einer Sperrklinke, einer Wippe oder einem ähnlichen Sperrelement arbeiten, das durch eine Dreh- oder Schwenkbewegung aus einem Sperrzustand in einen inaktiven Zustand versetzt wird. Bevorzugt weist der Sperrmechanismus jedoch einen Schieber auf, der bei einem Wechsel des Sperrmechanismus aus dem aktiven Zustand in den inaktiven Zustand verschoben wird. Ein solcher Schieber kann beispielsweise ein Sperrelement tragen, das den Antriebsmechanismus blockiert, insbesondere durch einen formschlüssigen Eingriff.

Möglich ist es auch, dass der bevorzugt bei einem erfindungsgemäßen Sperrmechanismus vorhandene Schieber selbst als Sperrelement durch formschlüssigen Eingriff in den Antriebsmechanismus oder in ein benutzerbetätigbares Betätigungselement eine Sperrung des Antriebsmechanismus bewirkt. An sich könnte ein solcher Schieber bei einem Wechsel des Sperrmechanismus aus dem aktiven Zustand in den inaktiven Zustand in einer beliebigen Richtung verschoben werden. Bevorzugt ist der Schieber aber in Stichrichtung verschiebbar, da dies einen kompakten Aufbau erleichtert.

Eine vorteilhafte Weiterbildung der Erfindung sieht vor, dass der Schieber federbeaufschlagt ist. Als Feder kann dabei ein beliebiges Bauteil verwendet werden, das bei Verformung eine Rückstellkraft erzeugt. Beispielsweise können als Feder ein elastisch verformbarer Kunststoffblock, eine Wendel aus Kunststoff oder Metall und ein elastisch verformbares Band, beispielsweise ein Gummiband, verwendet werden. Durch Einsatz eines federbeaufschlagten Schiebers lässt sich erreichen, dass dieser durch Federkraft zu seiner Anfangs- oder Endposition hinbewegt wird. Die Gefahr, dass der Schieber längere Zeit in einem undefinierten Zwischenzustand zwischen Anfangs- und Endposition verbleibt, lässt sich folglich reduzieren. Bevorzugt entspannt sich die Feder bei einem Wechsel des Sperrmechanismus in den inaktiven Zustand, gibt also zumindest einen Teil der in ihr gespeicherten Energie frei. Diese Maßnahme hat den Vorteil, dass ein Benutzer für das Deaktivieren des Sperrmechanismus keine zusätzliche Kraft aufbringen muss. Zudem lässt sich auf diese Weise ein besonders einfacher Aufbau des Sperrmechanismus erreichen, da es genügt im aktiven Zustand des Sperrmechanismus eine Verschiebung des Schiebers mit einem beweglichen Element, beispielsweise einem Anschlag oder einer Sperre, zu blockieren. Beim Ankoppeln der Insertionsvorrichtung an eine Basiseinheit kann ein solches Element durch Kontakt mit einem passenden Bauteil der Basiseinheit bewegt und so eine Verschiebung des Schiebers ermöglicht werden.

Bevorzugt ist der Schieber mit einem Verriegelungselement gekoppelt, das beim Ankoppeln der Insertionsvorrichtung an die Basiseinheit in eine Eingriffsposition geschoben wird, in der es die Insertionsvorrichtung formschlüssig mit der Basiseinheit verbindet. Auf diese Weise lässt sich erreichen, dass der Sperrmechanismus erst dann in seinen inaktiven Zustand übergeht, wenn die Insertionsvorrichtung formschlüssig und damit zuverlässig mit der Basiseinheit verbunden ist.

Bevorzugt weist der Sperrmechanismus ein Schutzelement auf, das sich in dem aktiven Zustand des Sperrmechanismus in Stichrichtung vor einer von dem Insertionsnadelhalter gehaltenen Insertionsnadel befindet. Diese Maßnahme hat den Vorteil, dass die Insertionsnadel abgedeckt und folglich die Verletzungsgefahr beim Handhaben der Insertionsvorrichtung noch weiter reduziert ist. Ein solches Schutzelement kann beispielsweise mit dem erwähnten Schieber verbunden sein, insbesondere gelenkig verbunden sein, so dass es bei einem Übergang des Sperrmechanismus in den inaktiven Zustand beiseite geschoben und so der Weg für eine Stichbewegung der Insertionsnadel freigegeben wird. Das Schutzelement, das beispielsweise plattenförmig ausgebildet sein kann, kann vorteilhaft mit dem erwähnten Verriegelungselement verbunden sein, insbesondere auch einstückig mit dem Verriegelungselement ausgebildet sein, das beim Ankoppeln der Insertionsvorrichtung einen Formschluss mit der Basiseinheit bewirkt.

Der Antriebsmechanismus einer erfindungsgemäßen Insertionsvorrichtung kann einen Energiespeicher, beispielsweise eine Feder, enthalten, um die für eine Stichbewegung erforderliche Energie zu liefern. Möglich ist es aber auch, dass der Antriebsmechanismus einer erfindungsgemäßen Insertionsvorrichtung im Betrieb eine Antriebsbewegung eines Betätigungselements in eine Stichbewegung des Insertionsnadelhalters umsetzt. Bevorzugt ist dabei, dass der Antriebsmechanismus im Anschluss an eine Stichbewegung eine Rückführbewegung des Insertionsnadelhalters bewirkt und nach abgeschlossener Rückführbewegung blockiert wird. Diese Blockade kann ebenfalls durch den Sperrmechanismus oder durch einen davon unabhängigen Mechanismus bewirkt werden. Beispielsweise ist es möglich, dass ein Betätigungselement, dessen Antriebsbewegung von dem Antriebsmechanismus in eine Stichbewegung umgesetzt wird, am Ende seines Betätigungswegs einrastet.

Weitere Einzelheiten und Vorteile der Erfindung werden an Ausführungsbeispielen unter Bezugnahme auf die beigefügten Zeichnungen beschrieben. Gleiche und einander entsprechende Bauteile sind dabei mit übereinstimmenden Bezugszahlen gekennzeichnet. Es zeigen:
- Figur 1: ein Ausführungsbeispiel einer erfindungsgemäßen Insertionsvorrichtung;
- Figur 2: ein Ausführungsbeispiel einer dazugehörenden Basiseinheit;
- Figur 3: einen Ausschnitt der in Figur 1 gezeigten Insertionsvorrichtung bei geöffnetem Gehäuse;
- Figur 4: eine Ansicht gemäß Figur 3 mit angekoppelter Basiseinheit;
- Figur 5: einen Ausschnitt eines weiteren Ausführungsbeispiels einer Insertionsvorrichtung bei geöffnetem Gehäuse; und
- Figur 6: eine Ansicht gemäß Figur 5 mit angekoppelter Basiseinheit.

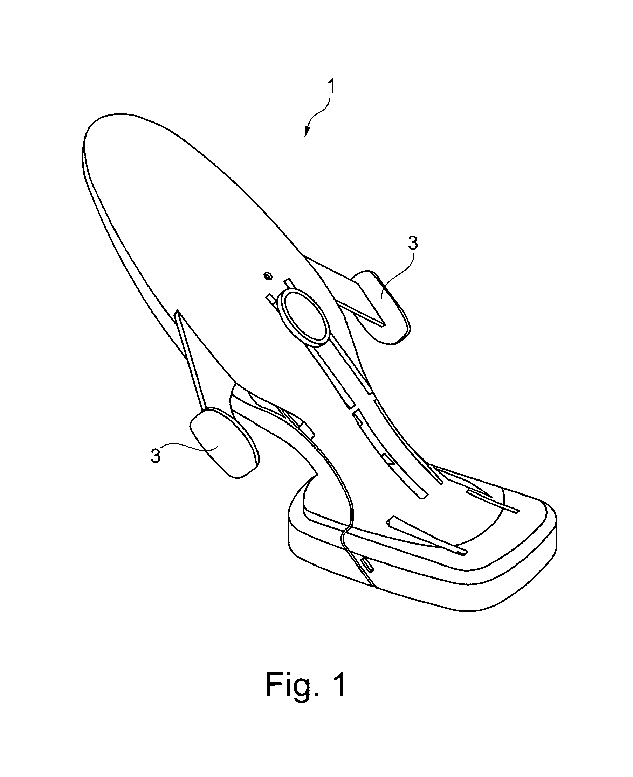

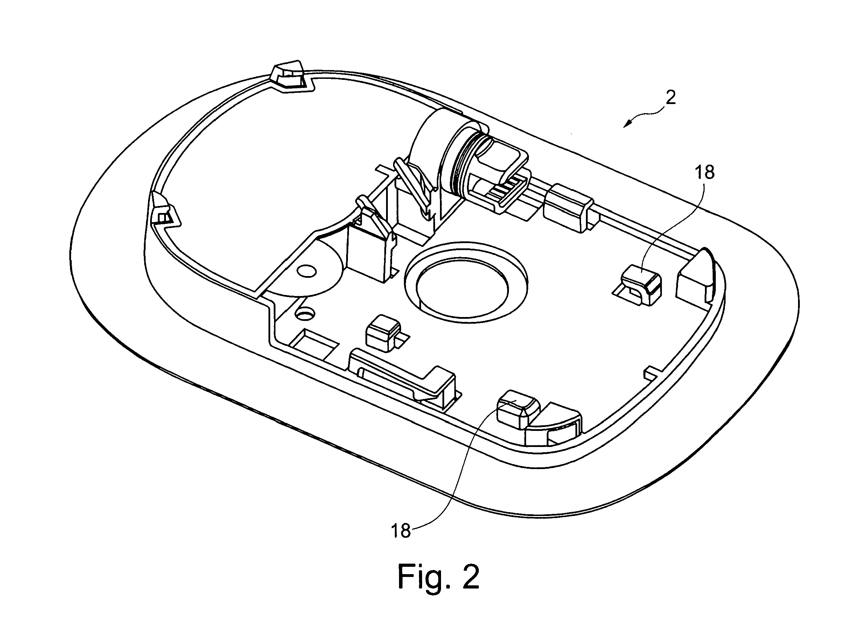

Die in Figur 1 gezeigte Insertionsvorrichtung 1 und die in Figur 2 gezeigte Basiseinheit 2 bilden zusammen ein Insertionssystem, mit dem beispielsweise Sensoren mittels Insertionsnadeln oder Katheter zur Infusion von Insulin oder anderen Wirkstoffen in den Körper eines Patienten insertiert werden können. Zur Insertion wird die Basiseinheit 2 mit ihrer Unterseite auf den Körper eines Patienten aufgeklebt und anschließend die Insertionsvorrichtung 1 an die Basiseinheit 2 angekoppelt.

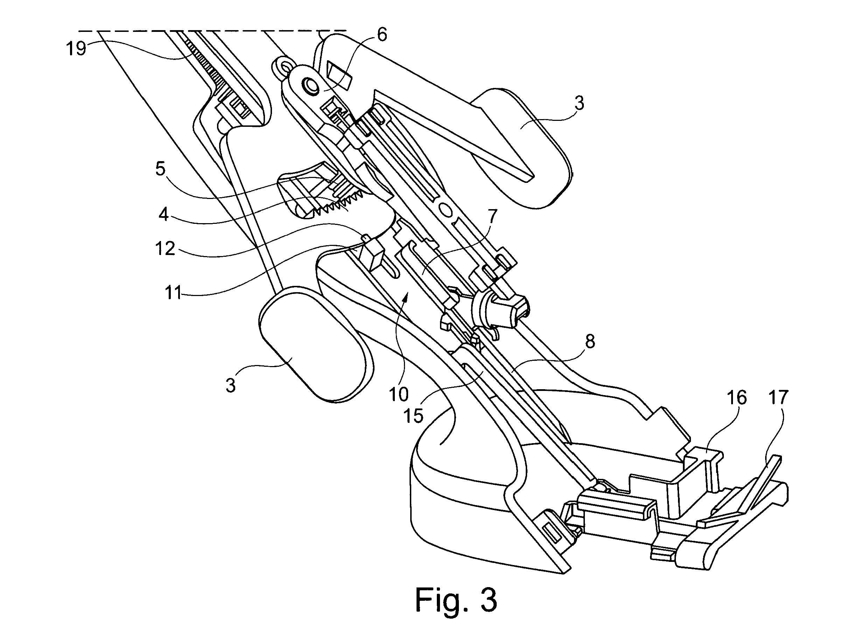

Die in Figur 1 gezeigte Insertionsvorrichtung 1 hat zwei Betätigungselemente 3, die für eine Insertion in einer Antriebsbewegung aufeinander zu bewegt werden. Diese Antriebsbewegung wird von einem in den Figuren 3 bis 6 gezeigten Antriebsmechanismus in eine Stichbewegung eines Insertionsnadelhalters und somit einer Insertionsnadel umgesetzt.

Wie Figur 3 zeigt, sind die Betätigungselemente 3 mit Zahnstangen 4 versehen, die beim Zusammendrücken der beiden Betätigungselemente 3 einen Rotor 5 in Drehung versetzen, die über einen Pleuel 6 in eine lineare Stichbewegung eines Insertionsnadelhalters 7 und einer von ihm getragene Insertionsnadel 8 umgesetzt wird. Die Zahnstangen 4, der Rotor 5 und der Pleuel 6 bilden zusammen den Antriebsmechanismus der Insertionsvorrichtung 1.

Um einer Verletzungsgefahr durch eine vorzeitige Stichbewegung zu begegnen, hat die Insertionsvorrichtung 1 einen Sperrmechanismus 10, der in einem aktiven Zustand eine Sperrung des Antriebsmechanismus bewirkt, also dessen Bewegung blockiert, und durch Ankoppeln der Insertionsvorrichtung 1 an die Basiseinheit 2 in einen inaktiven Zustand versetzt wird, in dem die Sperrung gelöst ist.

Bei dem in Figur 3 dargestellten Ausführungsbeispiel weist der Sperrmechanismus 10 einen Schieber 11 auf, der ein Sperrelement 12 trägt, das in dem in Figur 3 gezeigten aktiven Zustand in eine Ausnehmung 13 eines der Betätigungselemente 3 eingreift und so den Antriebsmechanismus blockiert. Der Schieber 11 ist gelenkig über einen Arm 15 mit einem Verriegelungselement 16 verbunden, das beim Ankoppeln der Insertionsvorrichtung 1 an die Basiseinheit 2 in eine Eingriffsposition geschoben wird, in der es die Insertionsvorrichtung 1 formschlüssig mit der Basiseinheit 2 verbindet. In dem in Figur 3 gezeigten aktiven Zustand des Sperrmechanismus wird eine Verschiebung des Verriegelungselements 16 durch ein Blockadeelement 17 verhindert.

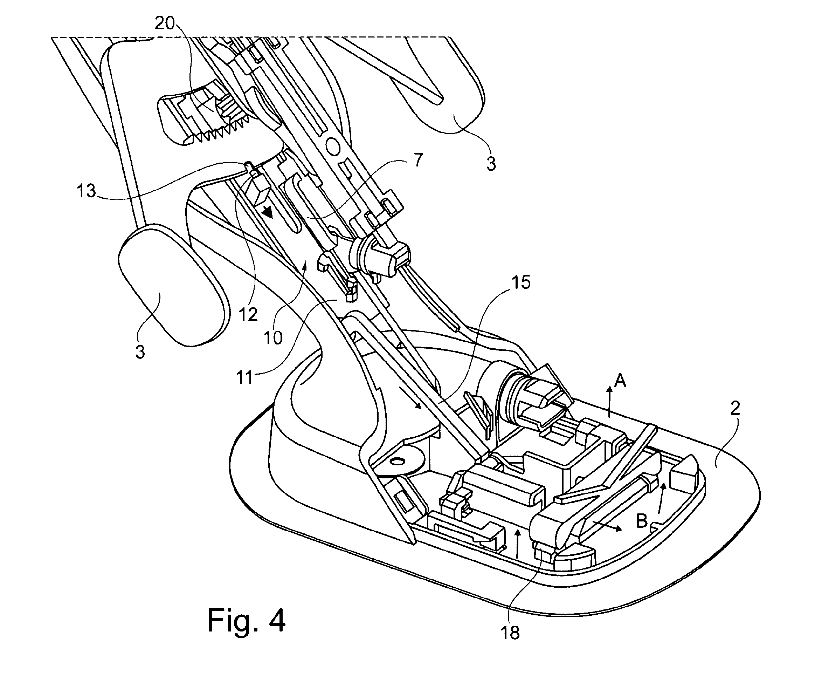

Beim Ankoppeln der Insertionsvorrichtung 1 an die Basiseinheit 2 wird das Blockadeelement 17 durch die Basiseinheit 2 quer zur Verschieberichtung des Verriegelungselements 16 bewegt, bei dem dargestellten Ausführungsbeispiel angehoben, und so ein in die Eingriffsposition führender Verschiebeweg für das Verriegelungselement 16 freigegeben. Durch eine anschließende Verschiebebewegung tritt das Verriegelungselement 16 mit der Basiseinheit 2 in Eingriff, nämlich in dem es unter dafür vorgesehene Eingriffselemente 18 geschoben wird, die insbesondere in Figur 2 und 4 zu sehen sind.

Diese Verschiebebewegung wird durch eine in Figur 3 dargestellte Feder 19 bewirkt, die bei dem dargestellten Ausführungsbeispiel als eine Wendelfeder, bevorzugt aus Kunststoff, ausgebildet ist und auf den Schieber 11 drückt. Die Feder 19 entspannt sich, wenn der Sperrmechanismus 11 aus dem in Figur 3 dargestellten aktiven Zustand in seinen in Figur 4 dargestellten inaktiven Zustand übergeht. Dabei verschiebt die Feder 19 den Schieber 11 in Stichrichtung. Diese Verschiebebewegung wird über den gelenkigen Arm 15 auf das Verriegelungselement 16 übertragen, das sich dadurch in seine Eingriffsposition in der Basiseinheit 2 bewegt.

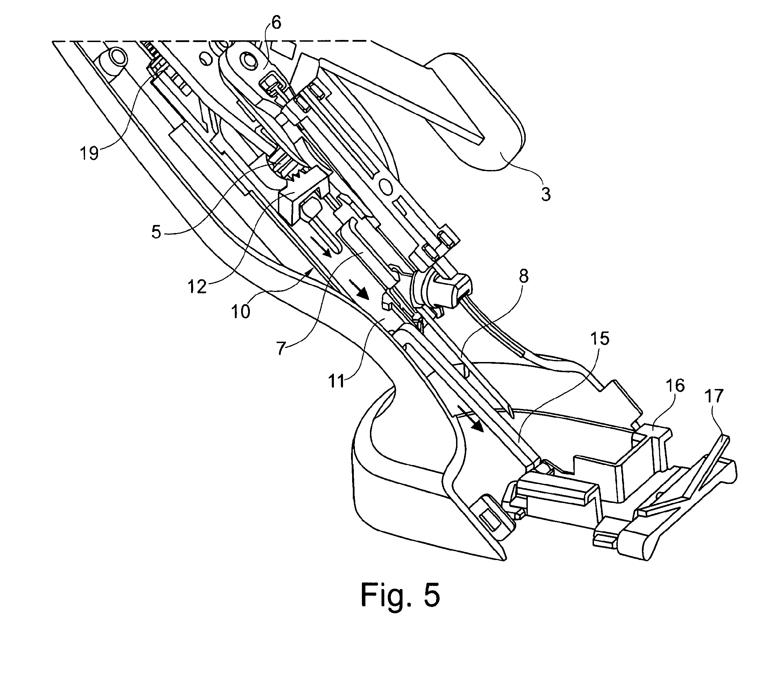

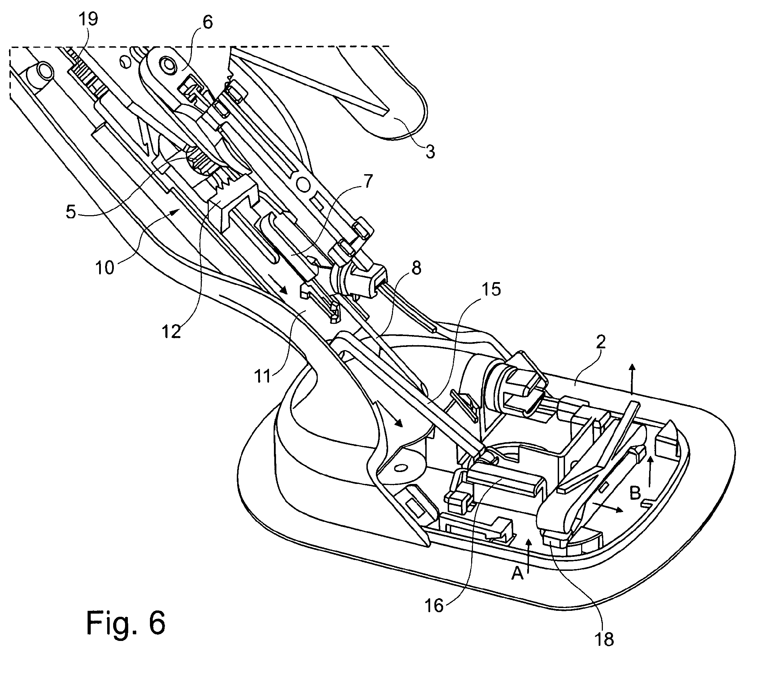

Die Figuren 5 und 6 zeigen ein weiteres Ausführungsbeispiel, das sich von dem vorstehend beschriebenen Ausführungsbeispiel im wesentlichen nur dadurch unterscheidet, dass ein von dem Schieber 11 getragenes Sperrelement 12 in dem in Figur 5 dargestellten aktiven Zustand des Sperrmechanismus 10 in den als Zahnrad ausgebildeten Rotor 5 des Antriebsmechanismus eingreift und diesen so blockiert. Durch Verschiebung in des Schiebers 11 in Stichrichtung geht der Sperrmechanismus in den in Figur 6 gezeigten inaktiven Zustand über.

Bei beiden Ausführungsbeispielen wird eine Verletzungsgefahr bei Handhabung der Insertionsvorrichtung 1 zusätzlich dadurch reduziert, dass ein Bauteil des Sperrmechanismus 10 sich in dem aktiven Zustand des Sperrmechanismus in Stichrichtung vor einer von dem Insertionsnadelhalter 7 gehaltenen Insertionsnadel 8 befindet. Bei den dargestellten Ausführungsbeispielen ist dieses als Schutzelement wirkende Bauteil eine Schiebeplatte, die zugleich das Verriegelungselement 16 bildet. Beim Übergang des Sperrmechanismus 10 in seinen inaktiven Zustand wird das Verriegelungselement 16 so verschoben, dass die Insertionsnadel 8 durch eine im Boden der Basiseinheit 2 vorgesehene Öffnung hindurch bewegt und so in den Körper eines Patienten eingestochen werden kann.

Der Schieber 11 des Sperrmechanismus 10 ist bei den beiden Ausführungsbeispielen jeweils in Stichrichtung verschiebbar und mit einer Linearführung versehen. Bei den dargestellten Ausführungsbeispielen ist die Linearführung als ein Schlitz in dem Schieber 11 ausgebildet, durch den ein Führungselement 20 des Gehäuses hindurch greift.

Die Betätigungselemente 3 können am Ende einer Antriebsbewegung mit einem nicht dargestellten Rastmechanismus verrasten, damit nach dem Abkoppeln einer Insertionsvorrichtung 1 von der Basiseinheit 2, also nach einer Insertion, eine unbeabsichtigte Stichbewegung, die zu einer Verletzung führen könnte, vermieden werden kann.

Der Antriebsmechanismus bewirkt im Anschluss an eine Stichbewegung eine Rückführbewegung des Insertionsnadelhalters 7. Bevorzugt wird durch diese Rückführbewegung die Kopplung zwischen Insertionsvorrichtung 1 und Basiseinheit 2 wieder gelöst. Dies kann beispielsweise dadurch erreicht werden, dass der Schieber 11 bei der Rückführbewegung an den Insertionsnadelhalter 7 koppelt und von ihm zurückgezogen wird. Beispielsweise kann der Schieber 11 ein schräg stehendes Federblättchen tragen, das bei der Stichbewegung von dem Insertionsnadelhalter umgebogen wird, so dass der Insertionsnadelnalter 7 über den Schieber 11 hinweg gleiten kann. Bei der Rückführbewegung kann ein solches Federblättchen in den Insertionsnadelhalter 7 eingreifen, so dass der Schieber 11 von dem Insertionsnadelhalter 7 zurückgezogen wird und folglich über den Arm 15 auch das Verriegelungselement 16 aus seiner Eingriffsposition mit der Basiseinheit 2 gezogen wird.

### Bezugszahlen

- 1: Insertionsvorrichtung
- 2: Basiseinheit
- 3: Betätigungselement
- 4: Zahnstange
- 5: Rotor
- 6: Pleuel
- 7: Insertionsnadelhalter
- 8: Insertionsnadel
- 9:
- 10: Sperrmechanismus
- 11: Schieber
- 12: Sperrelement
- 13: Ausnehmung
- 14:
- 15: Arm
- 16: Verriegelungselement
- 17: Blockadeelement
- 18: Eingriffselement
- 19: Feder
- 20: Führungselement

## Patentansprüche

1. Insertionssystem mit
einer Basiseinheit (2) zum Aufsetzen auf den Körper eines Patienten und
einer an die Basiseinheit (2) ankoppelbaren Insertionsvorrichtung (1),
wobei die Insertionsvorrichtung (1) einen Insertionsnadelhalter (7) zum Halten einer Insertionsnadel (8) und einen Antriebsmechanismus (4, 5, 6) zum Bewegen des Insertionsnadelhalters (7) in einer Stichrichtung aufweist,
**dadurch gekennzeichnet, dass** die Insertionsvorrichtung (1) einen Sperrmechanismus (10) aufweist, der in einem aktiven Zustand eine Sperrung des Antriebsmechanismus (4, 5, 6) bewirkt und durch Ankoppeln der Insertionsvorrichtung (1) an die Basiseinheit (2) in einen inaktiven Zustand versetzt wird, in dem die Sperrung gelöst ist.

2. Insertionsvorrichtung für ein Insertionssystem nach Anspruch 1, wobei die Insertionsvorrichtung (1) einen Insertionsnadelhalter (7) zum Halten einer Insertionsnadel (8) und einen Antriebsmechanismus (4, 5, 6) zum Bewegen des Insertionsnadelhalters (7) in einer Stichrichtung aufweist,
**gekennzeichnet durch** einen Sperrmechanismus (10), der in einem aktiven Zustand eine Sperrung des Antriebsmechanismus (4, 5, 6) bewirkt und **durch** Ankoppeln der Insertionsvorrichtung (1) an eine Basiseinheit (2) in einen inaktiven Zustand versetzt wird, in dem die Sperrung gelöst ist.

3. Insertionsvorrichtung nach Anspruch 2, **dadurch gekennzeichnet, dass** der Sperrmechanismus (10) einen Schieber (11) aufweist, der bei einem Wechsel des Sperrmechanismus (10) aus dem aktiven Zustand in den inaktiven Zustand verschoben wird.

4. Insertionsvorrichtung nach Anspruch 3, **dadurch gekennzeichnet, dass** der Schieber (11) in Stichrichtung verschiebbar ist.

5. Insertionsvorrichtung nach Anspruch 3 oder 4, **dadurch gekennzeichnet, dass** der Schieber (11) federbeaufschlagt ist.

6. Insertionsvorrichtung nach Anspruch 5, **dadurch gekennzeichnet, dass** sich die Feder (19) bei einem Wechsel des Sperrmechanismus (10) in den inaktiven Zustand entspannt.

7. lnsertionsvorrichtung nach einem der Ansprüche 3 bis 6, **dadurch gekennzeichnet, dass** der Schieber (11) mit einem Verriegelungselement (16) gekoppelt ist, das beim Ankoppeln der Insertionsvorrichtung (1) an die Basiseinheit (2) in eine Eingriffsposition geschoben wird, in der es die Insertionsvorrichtung (1) formschlüssig mit der Basiseinheit (2) verbindet.

8. Insertionsvorrichtung nach Anspruch 7, **dadurch gekennzeichnet, dass** in dem aktiven Zustand des Sperrmechanismus (10) ein Blockadeelement (17) eine Verschiebung des Verriegelungselements (16) blockiert.

9. Insertionsvorrichtung nach Anspruch 8, **dadurch gekennzeichnet, dass** das Blockadeelement (17) beim Ankoppeln der Insertionsvorrichtung (1) an die Basiseinheit (2) quer zur Verschieberichtung des Verriegelungselements (16) bewegt und so ein in die Eingriffsposition führender Verschiebeweg für das Verriegelungselement (16) freigegeben wird.

10. Insertionsvorrichtung nach einem der Ansprüche 7 bis 9, **dadurch gekennzeichnet,** der Schieber (11) über ein Gelenk mit dem Verriegelungselement (16) gekoppelt ist.

11. Insertionsvorrichtung nach einem der Ansprüche 2 bis 10, **dadurch gekennzeichnet, dass** der Sperrmechanismus (10) in dem aktiven Zustand ein Betätigungselement des Antriebsmechanismus (4, 5, 6) blockiert.

12. Insertionsvorrichtung nach Anspruch 11, **dadurch gekennzeichnet, dass** der Antriebsmechanismus (4, 5, 6) im Betrieb eine Antriebsbewegung des Betätigungselements (3) in eine Stichbewegung des Insertionsnadelhalters (7) umsetzt.

13. Insertionsvorrichtung nach einem der Ansprüche 3 bis 12, **gekennzeichnet durch** eine Linearführung für den Schieber (11).

14. Insertionsvorrichtung nach einem der Ansprüche 2 bis 13, **dadurch gekennzeichnet, dass** der Antriebsmechanismus (4 ,5 ,6) im Anschluss an eine Stichbewegung eine Rückführbewegung des Insertionsnadelhalters (7) bewirkt und nach abgeschlossener Rückführbewegung blockiert wird.

15. Insertionsvorrichtung nach einem der Ansprüche 2 bis 13, **dadurch gekennzeichnet, dass** der Sperrmechanismus (10) ein Schutzelement aufweist, das sich in dem aktiven Zustand des Sperrmechanismus (10) in Stichrichtung vor einer von dem Insertionsnadelhalter (7) gehaltenen Insertionsnadel (8) befindet.
